# EUROPEAN PATENT SPECIFICATION

(11) **EP 1 874 726 B1**
(45) Date of publication and mention of the grant of the patent: **16.05.2012**
(21) Application number: 06742574.4
(22) Date of filing: 12.04.2006
(51) Int. Cl.: C07C 279/18, C07C 277/08, A61K 9/19, A61K 31/24

(54) **ORGANIC COMPOUNDS**
ORGANISCHE VERBINDUNGEN
COMPOSES ORGANIQUES

(30) Priority: 14.04.2005 GB 0507577
(43) Date of publication of application: 09.01.2008
(60) Divisional application: 10161362.8
(73) Proprietor: Novartis AG, 4056 Basel (CH); IRM LLC, Hamilton, HM 11 (BM)
(72) Inventor: DANAHAY, Henry L., Horsham, West Sussex RH 12 5AB (GB); LEGRAND, Darren M., Horsham, West Sussex RH12 5AB (GB); TULLY, David, C., San Diego, CA 92130 (US); HARRIS, Jennifer, Leslie, San Diego, CA 92129 (US); HEUERDING, Silvia, CH-4051 Basel (CH); SINGH, Dilraj, CH-4058 Basel (CH); MAAS, Janet C., Horsham, West Sussex RH12 5AB (GB); ROETTELE, Juergen, 79112 Freiburg (DE); REBER, Jean-Louis, F-68680 Kembs (FR); MONNIER, Stéphanie, F-68190 Raedersheim (FR)
(74) Representative: Vögeli-Lange, Regina
(86) International application number: PCT/EP2006/003387
(87) International publication number: WO 2006/108643

(56) References cited:
- EP-A- 1 736 153
- EP-A2- 0 350 840
- US-A- 4 021 472
- US-A- 2002 142 956
- US-B1- 6 388 122
- DATABASE WPI Week 200379 Derwent Publications Ltd., London, GB; AN 2003-847969 XP002422321 & JP 2003 252754 A (TEIJIN LTD) 10 September 2003 (2003-09-10)
- DATABASE WPI Week 199807 Derwent Publications Ltd., London, GB; AN 1998-071864 XP002422322 & JP 09 309873 A (JUNSEI KAGAKU KK) 2 December 1997 (1997-12-02)

## Description

This invention relates to organic compounds, salts, formulation and processes and their use as pharmaceuticals.

US Patent application No. US 2002/142956 discloses camostat (free base) as an agent which stimulates secretin release and describes a method of treatment of cystic fibrosis which involves agents which trigger anion efflux via the activation of a secretin receptor.

The invention provides, in one aspect, the use of a serine protease inhibitor, camostat mesylate, for the preparation of a medicament for the treatment of a disease mediated by inhibition of a channel activating protease (CAP). The disease is suitably a respiratory disease, more suitably cystic fibrosis .

Examples of suitable serine proteases, the inhibitors of which are implicated in the uses of the present invention, include trypsin, matriptase, prostasin (PRSS8), plasmin, tPA, uPA, Xa, IXa, thrombin, tissue factor, compliment factors, tryptase, HNE, kallikrein (plasma and tissue), matriptase and TRMPSS 3 and 4.

The invention provides N,N-dimethyl-carbamoylmethyl-p-(p-guanidinobenzoyloxy)-phenylacetate, which has the non-proprietary name camostat, in the treatment or cystic fibrosis. Camostat mesylate (Foipan^{™}) is a particularly preferred form of camostat and is a known trypsin-like serine protease inhibitor that has been used to treat acute symptoms of chronic pancreatitis. This compound is prepared using the method described in Example 13 of United States patent specification US 4,021,472.

The serine protease inhibitor is preferably a synthetic trypsin-like serine protease inhibitor, especially a prostasin inhibitor such as camostat, or a suitable salt thereof.

Treatment of diseases mediated by inhibition of a channel activating protease in accordance with the invention may be prophylactic or symptomatic.

According to one aspect of the present invention, camostat mesylate and formulations thereof may be used in the treatment of diseases mediated by inhibition of a channel activating protease, especially by a trypsin-like serine protease, such as prostasin. Such diseases include diseases associated with the regulation of fluid volumes across epithelial membranes. For example, the volume of airway surface liquid is a key regulator of mucociliary clearance and the maintenance of lung health. The inhibition of a channel activating protease will promote fluid accumulation on the mucosal side of the airway epithelium thereby promoting mucus clearance and preventing the accumulation of mucus and sputum in respiratory tissues (including lung airways). Such diseases include respiratory diseases such as cystic fibrosis.

The suitability of a channel activating protease inhibitor, such as a prostasin inhibitor, for the treatment of a disease mediated by inhibition of a channel activating protease, may be tested by determining the inhibitory effect of the channel activating protease inhibitor on: (1) the native, isolated, purified or recombinant channel activating protease using a suitable biochemical assay format using the method described in Shipway et al. Biochemical and Biophysical Research Communications 2004; 324(2):953-63), and/or (2) the ion channel / ion transport function in suitable isolated cells or confluent epithelia using the methods described in Bridges et al., American Journal of Physiology Lung Cell Molecular Physiology 2001;281(1):L16-23) and Donaldson et al., Journal of Biological Chemistry 2002;277(10):8338-45).

Channel activating protease inhibitors, especially camostat mesylate are also useful as co-therapeutic agents for use in combination with other drug substances such as anti-inflammatory, bronchodilatory, antihistamine or anti-tussive drug substances, particularly in the treatment of cystic fibrosis or obstructive or inflammatory airways diseases such as those mentioned hereinbefore, for example as potentiators of therapeutic activity of such drugs or as a means of reducing required dosaging or potential side effects of such drugs.

Camostat mesylate for use in the present invention may be mixed with the other drug substance in a fixed pharmaceutical composition or it may be administered separately, before, simultaneously with or after the other drug substance.

Accordingly the invention includes a combination of camostat mesylate with an anti-inflammatory, bronchodilatory, antihistamine, anti-tussive, antibiotic or DNase drug substance, said channel activating protease inhibitor and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in international patent application WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (Ariflo® GlaxoSmithKline), Roflumilast (Byk Gulden), V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; and adenosine A_{2B} receptor antagonists such as those described in WO 02/42298.

Suitable bronchodilatory drugs include beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound (5-[(R)-2-(5,6-Diethyl-indan-2-ylamino)-1-hydroxy-ethyl]-8-hydroxy-1H-quinolin-2-one) and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964.

Suitable bronchodilatory drugs also include anticholinergic or antimuscarinic agents, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285.

Suitable dual anti-inflammatory and bronchodilatory drugs include dual beta-2 adrenoceptor agonist / muscarinic antagonists such as those disclosed in US 2004/0167167, WO 04/74246 and WO 04/74812.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/099807 and WO 04/026841.

Suitable antibiotics include macrolide antibiotics, for example tobramycin (TOBI™).

Suitable DNase drug substances include dornase alfa (Pulmozyme™), a highly purified solution of recombinant human deoxyribonuclease I (rhDNase), which selectively cleaves DNA. Dornase alfa is used to treat cystic fibrosis.

Further suitable combinations with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methyl-phenyl)-5H-benzo-cyclohepten-8-yl]carbonyl]amino]phenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.

Camostat mesylate may also be combined with inhaled osmolytes, such as hypertonic saline, mannitol or dextran.

Camostat mesylate may also be combined with P2Y2 antagonists or agonists, CLC2 activators, ENaC inhibitors or PDE-5 inhibitors.

Camostat mesylate may also be combined with an NSAID, such as ibuprofen.

Camostat mesylate may be administered by any appropriate route, for example orally, e.g. in tablet, capsule or liquid form, parenterally, for example in the form of an injectable solution or suspension, or intranasally, for example in the form of an aerosol or other atomisable formulation using an appropriate intranasal delivery device, e.g. a nasal spray such as those known in the art, or by inhalation, which is preferred, especially for use with a nebulizer.

The camostat mesylate may be administered in a pharmaceutical composition together with a pharmaceutically acceptable diluent or carrier. Such compositions may be, for example dry powders, tablets, capsules and liquids, but also injection solutions, infusion solutions or inhalation powders, aqueous and propellant based, solutions or suspensions, which may be prepared using other formulating ingredients and techniques known in the art.

The dosage of the camostat mesylate can depend on various factors, such as the activity and duration of action of the active ingredient, the severity of the condition to be treated, the mode of administration, the species, sex, ethnic origin, age and weight of the subject and/or its individual condition. In a normal case the daily dose for administration, for example oral administration, to a warm-blooded animal, particularly a human being, weighing about 75 kg is estimated to be from approximately 0.7 mg to approximately 1400 mg, especially from approximately 5 mg to approximately 200 mg. That dose may be administered, for example, in a single dose or in several part doses of, for example, from 5 to 200 mg.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the channel activating protease inhibitor having a particle diameter up to 10 microns, optionally together with a diluent or carrier, such as lactose, of the desired particle size distribution and a compound that helps to protect against product performance deterioration due to moisture e.g. magnesium stearate. When the composition comprises a nebulised formulation, it preferably contains, for example, the channel activating protease inhibitor either dissolved, or suspended, in a vehic!e containing water, a co-solvent such as ethanol or propylene glycol and a stabiliser, which may be a surfactant.

The invention includes (A) camostat mesylate in inhalable form, e.g. in an aerosol or other atomisable composition or in inhalable particulate, e.g. micronised, form, (B) an inhalable medicament comprising camostat mesylate in inhalable form; (C) a pharmaceutical product comprising camostat mesylate in inhalable form in association with an inhalation device; and (D) an inhalation device containing camostat mesylate in inhalable form.

In another embodiment, the inhalation route of administration is in a powder form. The active ingredient may be used as a powder with a particle size of 0.5 to 10 micrometers, preferably 0.5-5 micrometers which can be produced by a variety of conventional techniques, such as jet-milling, spray-drying, solvent precipitation, and the like. One widely used formulation approach is to mix the fine active drug particles with a coarse bulking powder with a particle size of 10 to 500µm. The bulking powder is selected from saccharides, preferably lactose, sucrose, glucose, galactose, fructose, trehalose and raffinose, most preferably lactose. Preferably the particle size of the finely-divided solid powder should for physiological reasons be less than 25 microns and preferably less than about 10 microns in diameter. The particle size of the powder for inhalation therapy should most preferably be in the range of 2 to 10 microns.

Other embodiments of the present the invention include aerosol formulations which comprise the active ingredient suspended or dissolved in a suitable aerosol propellant, such as a chlorofluorocarbon (CFC) or a hydrofluorocarbon (HFC). Suitable CFC propellants include trichloromonofluoromethane (propellant 11), dichlorotetrafluoromethane (propellant 114), and dichlorodifluoromethane (propellant 12). Suitable HFC propellants include tetrafluoroethane (HFC-134a) and heptafluoropropane (HFC-227). The propellant comprises 40 to 90% by weight of the total inhalation composition. A yet further suitable propellant is ethanol.

Preferably, for incorporation into the aerosol propellant, the active ingredient, e.g. camostat or a pharmaceutically acceptable salt thereof, will be processed into respirable particles as described for the dry powder formulations. The particles are then suspended in the propellant, typically being coated with a surfactant to enhance their dispersion properties. Such surfactants include sorbitan tiroleate, oleyl alcohol, oleic acid, lecithin or oils derived from natural sources, such as, corn oil, olive oil, cotton seed oil and sunflower seed oil are useful in keeping the suspended particles form agglomerating.

As a further aspect of the invention, camostat mesylate for use in the present invention may be prepared as a formulation suitable for nebulisation. Such formulations contain excipients physiologically compatible with the bronchial epithelium, usually consisting of co-solvents, preservatives, chelating agents, pH and tonicity regulators and surfactants. Suitable excipients include but are not limited to, propylene glycol, ethanol, glycerin, benzalkonium chloride, sodium edentate, ascorbic acid, citric acid, hydrochloric acid, sodium hydroxide, sodium chloride, oleic acid, and lecithin. The nebulised formulation may be prepared by standard manufacturing techniques.

As a further aspect of the invention, camostat mesylate for use in the present invention, may be prepared in a lyophilised form for subsequent reconstitution in a physiologically acceptable vehicle for inhalation immediately prior administration. The lyophilised formulation may include the following optional additional excipients, lactose, mannitol and glucose. The lyophilised product may be prepared by an aseptic process first involving dissolving the active ingredient and excipients in an aqueous vehicle. The resulting solution is then filled in glass vials and subsequently freeze dried. The lyophilized camostat suitably includes lactose, suitably in a ratio of camostat or a salt form:lactose of 1:1 to 1:10.

As a further aspect of the invention, a formulation for use in the present invention, preferably a nebulized formulation of camostat mesylate, may be taste-masked by various approaches including addition of suitable further excipients either in the solution formulation or as an ingredient in the lyophilized form or vehicle for reconstitution. Suitable excipients for taste-masking according to the invention include but not limited to, saccharine sodium, aspartame, menthol and polyalcohols such as sorbitol and xylitol. The taste-masked formulation may be prepared by known and standard techniques, e.g. according to the methods described in WO2005/037246 and Manfred Keller, Antonio Manuel Fernandes Raposo et al. "Taste masking of a pentoxifylline formulation for pulmonary application (patent application). The taste-masked formulation of camostat suitably comprises sachharine sodium, suitably in a camostat or salt form: saccharine sodium ratio of 100:1 to 1:5.

### Biological Examples

The channel activating protease inhibitor, camostat mesylate, inhibits prostasin (human and guinea pig) and matriptase (human), attenuates the amiloride-sensitive, ENaC-mediated short circuit current in cultured human bronchial epithelial cells, and attenuates the tracheal potential difference in the guinea pig

*Biochemical assays:* Recombinant human prostasin and matriptase and guinea pig prostasin are generated according to methods described in Shipway et al., Biochemical and Biophysical Research Communications 2004; 324(2):953-63). The recombinant enzymes are incubated in an electrolyte buffer containing the test compounds or vehicle in a suitable multiple well assay plate such as a 96 or 384 well plate. At a defined time -after the mixing of enzyme with compound or vehicle, a suitable fluorescent peptide substrate is added to the assay mixture. As substrate becomes cleaved by the active enzyme, fluorescence (measured using a suitable fluorescence plate reader) increases and the rate of turnover of substrate (i.e. enzyme activity) can be quantified and thus the inhibitory effect of any test compound. The efficacy of test compounds is expressed as the concentration that induces a 50% attenuation in the enzyme activity (Kᵢ).

*Epithelial ion transport:* Human bronchial epithelial cells are cultured according to methods described in Danahay et al., American Journal of Physiology Lung Cell Molecular Physiology 2002; 282(2):L226-36). When suitably differentiated (days 14-21 after establishing an apical-air interface) epithelial cells are treated with either vehicle, aprotinin (200 µg/ml) or test compound for 90 minutes. Epithelia are then placed into Ussing Chambers as described in Danahay et al., American Journal of Physiology Lung Cell Molecular Physiology 2002; 282(2):L226-36) maintaining the concentration of vehicle, aprotinin or test compound on the apical side of the epithelia. Short circuit current (ISC) is then measured by voltage clamping the epithelia to zero millivolts. The amiloride-sensitive ISC is then measured by the addition of amiloride (10 µM) to the apical surface of the epithelia. The potency of the test compound is expressed as the concentration inducing a 50% inhibition of the total aprotinin-sensitive component of the amiloride-sensitive ISC.

*Tracheal potential difference (in vivo):* Guinea pigs are anaesthetized using a short acting inhalation anaesthesia such as halothane and N₂0. Whilst under short acting anaesthesia an oral gavage needle is inserted into the trachea via the oropharangeal route. Once inside the trachea, a small volume (50-200 µl) of vehicle or test compound, in a suitable aqueous-based diluent, is instilled into the airways. Animals then recover and become fully ambulatory. Alternatively test compounds can be administered to animals using aerosol or dry powder dosing. At a defined time after dosing, the animals are surgically anaesthetized using a suitable anaesthesia such as ketamine and xylazine. The trachea is then exposed and a plastic agar bridge electrode is inserted into the tracheal lumen. A reference electrode is also inserted into the layers of muscle in the animal's neck. The tracheal potential difference is then measured using a suitable high impedance voltmeter as described in Takahashi et al., Toxicol Appl Pharmacol. 1995; 131(1):31-6. The potency of the test compound is expressed as the dose inducing a 50% reduction in the sensitive-component of the tracheal potential difference.

The results are shown in the tables 1 and 2 below:

**Table 1 Summary of in vitro & in vivo data with Camostat mesylate**

| rh-prostasin (Ki) | rh-matriptase (Ki) | rGPig-prostasin (Ki) | HBEC (IC₅₀) | GPig tracheal PD in vivo (ED₅₀) |
|---|---|---|---|---|
| 0.429 µM | 0.005 µM | 0.098 µM | 0.05 µM | 2 hr = 3.3 µg/kg (i.t.) |
| | | | | 5 hr = 26 µg/kg (i.t.) |

**Table 2 Effects of Camostat mesylate on Guinea pig tracheal potential difference in vivo**

| | Camostat mesylate dose (µg/kg) by intra-tracheal administration | | | | | | | |
|---|---|---|---|---|---|---|---|---|
| | 0 | 1 | 3 | 10 | 30 | 100 | 300 | 1000 |
| 2 hr | -10.3 | -10.0 | -7.3 | -6.4 | -5.3 | -4.7 | | |
| | ±0.6 | ±0.7 | ±0.5 | ±0.5 | ±0.3 | ±0.5 | | |
| 5 hr | -9.9 | | -10.8 | -9.7 | -7.5 | -6.5 | -5.2 | -5.7 |
| | ±0.6 | | ±1.0 | ±1.5 | ±0.4 | ±0.3 | ±0.3 | ±0.4 |

| | | | | | | | | |
|---|---|---|---|---|---|---|---|---|
| Mean absolute values of the tracheal potential difference (± s.e.mean) are shown at either 2 or 5 hours after intra-tracheal dosing with vehicle or camostat mesylate. | | | | | | | | |

### Pharmaceutical Formulation Examples

The following are non-limiting examples of formulations of the present invention suitable for nebulisation. For the avoidance of doubt, 'camostat' as used below refers to camostat mesylate.

### Example 1

Camostat 25mg
Lactose 125mg
sodium saccharine 25mg
reconstitution with 5ml isotonic saline

### Example 2

Camostat 2.5mg
Lactose 125mg
sodium saccharine 2.5mg
reconstitution with 5ml isotonic saline

### Example 3

Camostat 25mg
Lactose 125mg
reconstitution with 5ml isotonic saline + sodium saccharine 25mg

### Example 4

Camostat 2.5mg
Lactose 125mg
reconstitution with 5ml isotonic saline + sodium saccharine 2.5mg

## Claims

1. Camostat mesylate, or a solvate thereof, for the treatment of cystic fibrosis.

2. A process for forming a lyophilised form of camostat mesylate according to claim 1 comprising (i) forming an aqueous solution comprising camostat mesylate, wherein the aqueous solution includes an excipient selected from lactose, mannitol and glucose and (ii) subjecting the aqueous solution to freeze-drying.

3. A process for forming a nebuliser formulation of camostat mesylate according to claim 1 comprising the process according to claim 2 and the additional step (iii) reconstituting the lyophilised form in an aqueous vehicle.

4. A taste-masked formulation comprising camostat mesylate and a taste-masking excipient.

5. A taste-masked formulation according to claim 4, including one or more excipients selected from saccharine sodium, aspartame, menthol and polyalcohols such as sorbitol and xylitol.

6. A taste-masked formulation according to claim 4 or 5 in the manufacture of a medicament for the treatment of cystic fibrosis.

7. A nebuliser formulation comprising camostat mesylate according to claim 1 and one or more excipients selected from co-solvents, preservatives, chelating agents, pH and tonicity regulators and surfactants.

8. A nebulizer formulation according to claim 7 where the excipients are selected from one or more of propylene glycol, ethanol, glycerin, benzalkonium chloride, sodium edentate, ascorbic acid, citric acid, hydrochloric acid, sodium hydroxide, sodium chloride, oleic acid, and lecithin.

9. A nebuliser formulation according to claim 7 or 8 further comprising one or more taste-masked excipients selected from saccharine sodium, aspartame, menthol and polyalcohols such as sorbitol and xylitol.

10. A nebuliser formulation according to any one of claims 7-9 for the treatment of cystic fibrosis.

## Patentansprüche

1. Camostatmesylat oder ein Solvat davon zur Behandlung von zystischer Fibrose.

2. Verfahren zur Bildung einer lyophilisierten Form von Camostatmesylat nach Anspruch 1, bei dem man (i) eine Camostatmesylat umfassende wässrige Lösung bildet, wobei die wässrige Lösung einen aus Lactose, Mannit und Glucose ausgewählten Exzipienten umfasst, und (ii) die wässrige Lösung einer Gefriertrocknung unterzieht.

3. Verfahren zur Bildung einer Verneblerformulierung von Camostatmesylat nach Anspruch 1, welches das Verfahren nach Anspruch 2 und den zusätzlichen Schritt (iii) der Rekonstitution der lyophilisierten Form in einem wässrigen Vehikel umfasst.

4. Geschmacksmaskierte Formulierung, umfassend Camostatmesylat und einen geschmacksmaskierenden Exzipienten.

5. Geschmacksmaskierte Formulierung nach Anspruch 4, die einen oder mehrere Exzipienten ausgewählt aus Saccharin-Natrium, Aspartam, Menthol und Polyalkoholen wie Sorbit und Xylit umfasst.

6. Geschmacksmaskierte Formulierung nach Anspruch 4 oder 5 bei der Herstellung eines Medikaments zur Behandlung von zystischer Fibrose.

7. Verneblerformulierung, umfassend Camostatmesylat nach Anspruch 1 und einen oder mehrere Exzipienten ausgewählt aus Cosolventien, Konservierungsstoffen, Chelatbildnern, den pH-Wert und die Tonizität einstellenden Mitteln und Tensiden.

8. Verneblerformulierung nach Anspruch 7, wobei es sich bei den Exzipienten um einen oder mehrere Exzipienten aus der Gruppe Propylenglykol, Ethanol, Glycerin, Benzalkoniumchlorid, Natriumedentat, Ascorbinsäure, Citronensäure, Salzsäure, Natriumhydroxid, Natriumchlorid, Ölsäure und Lecithin handelt.

9. Verneblerformulierung nach Anspruch 7 oder 8, welche weiterhin einen oder mehrere geschmacksmaskierte Exzipienten ausgewählt aus Saccharin-Natrium, Aspartam, Menthol und Polyalkoholen wie Sorbit und Xylit umfasst.

10. Verneblerformulierung nach einem der Ansprüche 7 bis 9 zur Behandlung von zystischer Fibrose.

## Revendications

1. Mésylate de camostat, ou un solvat de celui-ci, destiné au traitement de la fibrose kystique.

2. Procédé de formation d'une forme lyophilisée de mésylate de camostat selon la revendication 1, comprenant les étapes consistant à (i) former une solution aqueuse comprenant du mésylate de camostat, où la solution aqueuse comporte un excipient choisi parmi le lactose, le mannitol et le glucose, et (ii) soumettre la solution aqueuse à une lyophilisation.

3. Procédé de formation d'une formulation pour nébuliseur de mésylate de camostat selon la revendication 1, comprenant le procédé selon la revendication 2 et l'étape supplémentaire consistant à (iii) reconstituer le forme lyophilisée dans un véhicule aqueux.

4. Formulation à goût masqué comprenant du mésylate de camostat et un excipient masqueur de goût.

5. Formulation à goût masqué selon la revendication 4, comprenant un ou plusieurs excipients choisis parmi la saccharine de sodium, l'aspartame, le menthol et des polyalcools tels que le sorbitol et le xylitol.

6. Formulation à goût masqué selon la revendication 4 ou 5, dans l'élaboration d'un médicament destiné au traitement de la fibrose kystique.

7. Formulation de nébuliseur comprenant du mésylate de camostat selon la revendication 1 et un ou plusieurs excipients choisis parmi les co-solvants, les conservateurs, les agents chélateurs, les régulateurs de pH et de tonicité et les agents tensioactifs.

8. Formulation de nébuliseur selon la revendication 7, dans laquelle les excipients sont choisis parmi un ou plusieurs parmi le propylène glycol, l'éthanol, le glycérol, le chlorure de benzalkonium, l'édétate de sodium, l'acide ascorbique, l'acide citrique, l'acide chlorhydrique, l'hydroxyde de sodium, le chlorure de sodium, l'acide oléique et la lécithine.

9. Formulation de nébuliseur selon la revendication 7 ou 8, comprenant en outre un ou plusieurs excipients masqueurs de goût choisis parmi la saccharine de sodium, l'aspartame, le menthol et des polyalcools tels que le sorbitol et le xylitol.

10. Formulation de nébuliseur selon l'une quelconque des revendications 7-9, destinée au traitement de la fibrose kystique.
